# EUROPEAN PATENT APPLICATION

(11) **EP 1 731 522 A1**
(43) Date of publication of application: **13.12.2006**
(21) Application number: 05721621.0
(22) Date of filing: 28.03.2005
(51) Int. Cl.: C07D 493/14, C07B 53/00

(54) **PROCESS FOR PRODUCING FLAVONE C GLYCOSIDE DERIVATIVES**

(30) Priority: 31.03.2004 JP 2004107760
(71) Applicant: SUNTORY LIMITED, Osaka-shi, Osaka 530-8203 (JP)
(72) Inventor: TSUJI, Kuniro, Shizuoka, 422-8005 (JP); NUKAYA, Haruo;, Shizuoka-shi, Shizuoka; 424-0885 (JP)
(74) Representative: Vossius & Partner
(86) International application number: PCT/JP2005/005695
(87) International publication number: WO 2005/095416

(57) **Abstract**

The present invention provides processes of efficiently producing a flavone C-glycoside derivative represented by the formula (1) or a salt thereof and a flavone C-glycoside derivative represented by the formula (4) or a salt thereof that are substances having an anti-allergic effect. According to the present invention, the flavone C-glycoside derivative represented by the formula (1) or the salt thereof and the flavone C-glycoside derivative represented by the formula (4) or the salt thereof can easily be synthesized in good yield with isovitexin and vitexin contained in herbs or the like as raw materials.

## Description

### TECHNICAL FIELD

The present invention relates to a process of producing a novel flavone C-glycoside derivative or a salt thereof exhibiting an anti-allergic effect.

### BACKGROUND ART

Various epidemiological surveys have revealed that allergic diseases have been increasing in recent years. In particular, pollinosis and atopic dermatitis are markedly increasing, even to the extent that they have become a social issue. Such an increase in allergies is presumed to be caused by not only an increase in allergens but also by air pollution, food additives, and changes in environment, such as changes in diet (Ito, Sogo Rinsho in Japanese (Comprehensive Clinic) 41: 3099, 1992).

Of these allergic reactions, type I and type IV allergic reactions are considered to be involved in pollinosis and atopic dermatitis, for which anti-histamic drugs, basic anti-allergic drugs, steroid drugs, or the like are used in treatments.

On the other hand, it has been reported that a flavone C-glycoside derivative represented by the formula (1):

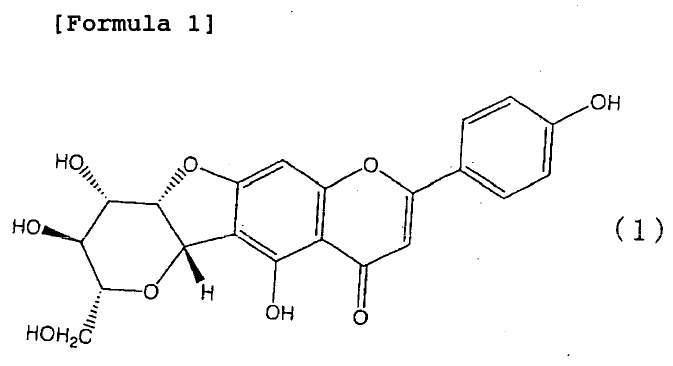

or a salt thereof or a flavone C-glycoside derivative represented by the formula (4):

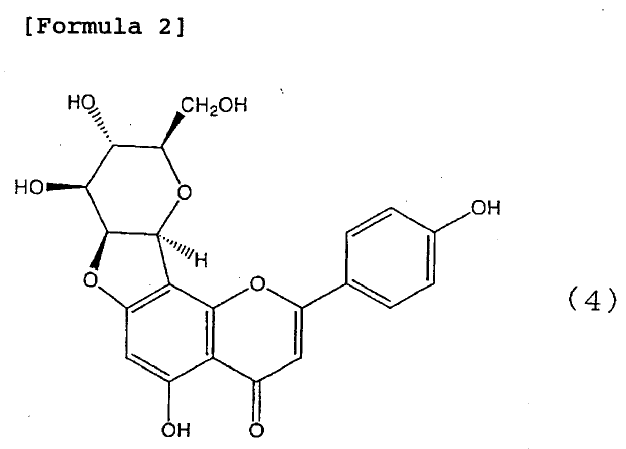

or a salt thereof, contained in oolong tea has an inhibitory effect on the formation of auricular edema induced by 2,4-dinitrofluorobenzen (DNFB) and an inhibitory effect on histamine release from peritoneal mast cells (Japanese Patent Laid-Open No. 2004-035474), and expectations are placed on their application to therapeutic agents for diseases associated with allergic reactions or to food additives. A feature of those compounds is that they are effective in small doses. Those compounds can be obtained by subjecting a tea leaf used as a raw material to extraction by heating and with a solvent, concentration, and general chemical separation/purification means such as purification, (e.g., fractionation or chromatography).

### DISCLOSURE OF THE INVENTION

An object of the present invention is to provide processes of efficiently producing a flavone C-glycoside derivative represented by the formula (1) (hereinafter, also referred to as a compound represented by the formula (1)) or a salt thereof and a flavone C-glycoside derivative represented by the formula (4) (hereinafter, also referred to as a compound represented by the formula (4)) or a salt thereof that are substances having an anti-allergic effect. The present invention is particularly intended to provide processes of efficiently producing a flavone C-glycoside derivative represented by the formula (1) or a salt thereof and a flavone C-glycoside derivative represented by the formula (4) or a salt thereof using easily-available raw materials.

The present inventor has diligently studied processes of efficiently producing a flavone C-glycoside derivative represented by the formula (1) or a salt thereof and a flavone C-glycoside derivative represented by the formula (4) or a salt thereof that are substances having an anti-allergic effect, and has paid attention to isovitexin and vitexin contained in herbs and the like. The present inventor has completed the present invention by finding that isovitexin and vitexin can be used as raw materials to easily synthesize, in good yield, a flavone C-glycoside derivative represented by the formula (1) and a flavone C-glycoside derivative represented by the formula (4), respectively, and that the unreacted raw materials are collected and recycled to thereby allow more efficient synthesis of the final product.

The present invention thus relates to a process of producing a flavone C-glycoside derivative represented by the formula (1):

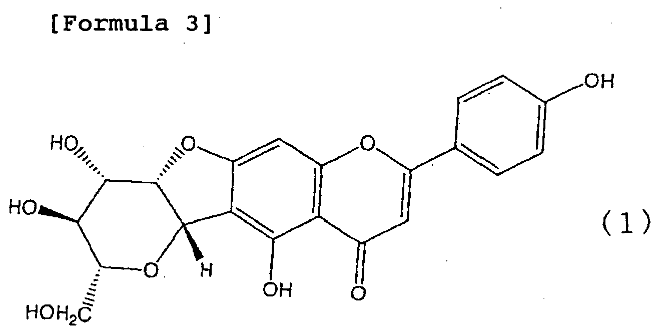

or a salt thereof.

The present invention also relates to a process of producing a flavone C-glycoside derivative represented by the formula (4):

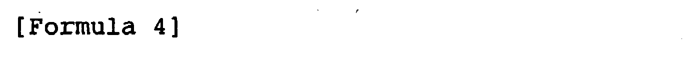

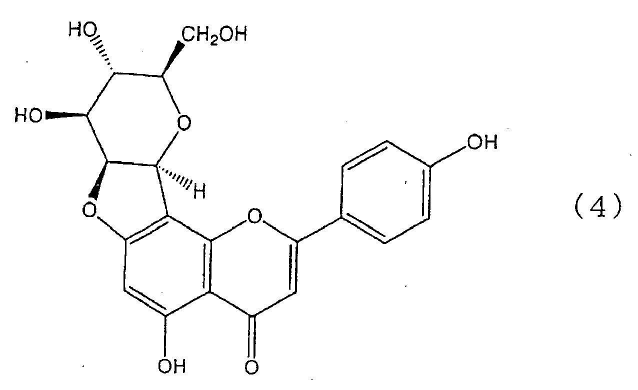

or a salt thereof.

### BEST MODE FOR CARRYING OUT THE INVENTION

### Raw material

A flavone C-glycoside derivative represented by the formula (1) or a salt thereof is produced with isovitexin as a raw material. Also, a flavone C-glycoside derivative represented by the formula (4) or a salt thereof is produced with vitexin as a raw material.

The structural formulas of isovitexin and vitexin are shown in the formula (5) and the formula (6), respectively.

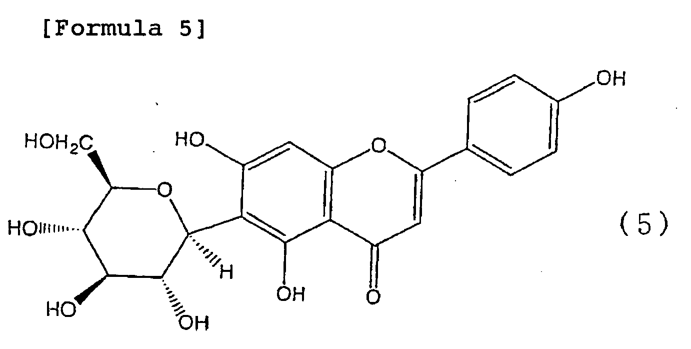

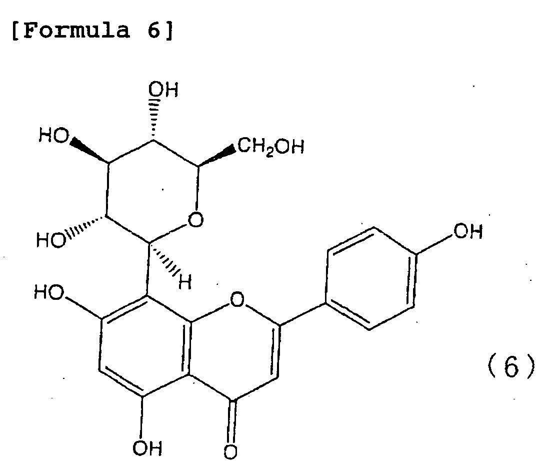

Isovitexin and vitexin are components contained in many plants, including herbs and buckwheat husks, and can be obtained by the steps of solvent extraction and chemical separation/purification. Among plants, there are also plants containing several percentage of isovitexin and vitexin which are thus easily available. Although a solvent used in elution may be any of aqueous solvents and/or organic solvents, the aqueous solvent is preferably used. An example of preferred aqueous solvents is water, ethanol, or methanol. The solvent may be water alone or an arbitrary mixed solution of water with a lower alcohol such as methanol or ethanol.

Although the proportion of a plant such as an herb to a solvent for extraction is not particularly limited, preferred is the proportion of 1 part by weight of the plant to 2 to 1000 parts by weight of the solvent, particularly 5 to 100 parts by weight of the solvent in light of extraction procedures and efficiency. A temperature for extraction is not particularly limited as long as it is higher than the melting point of a solvent and lower than the boiling point thereof. However, the preferred temperature is from 10°C to 100°C for water and from 10°C to 40°C for ethanol and methanol. It is preferred that the extraction time is set within the range of from 10 seconds to 24 hours.

The chemical separation/purification means that can be used is any of methods generally used as chemical separation/purification approaches; for example, liquid-liquid partition chromatography, thin-layer chromatography, adsorption column chromatography, partition column chromatography, gel filtration column chromatography, ion-exchange column chromatography, electrophoresis, and high-performance liquid chromatography. These separation/purification means can also be combined, if necessary, to highly purify isovitexin and vitexin used as raw materials.

Although isovitexin and vitexin used as raw materials are not necessarily required to be highly purified, isovitexin and vitexin of higher purity is preferred in light of reaction efficiency and the purification efficiency of a product.

For isovitexin and vitexin used as raw materials, hydroxyl groups other than those directly involved in water-eliminating condensation may be protected by protecting groups. It is considered that the addition of protecting groups reduces side reactions other than those of interest and improves the solubility of isovitexin and vitexin in reaction solvents. The types of protecting groups that can be used are those generally used as hydroxyl protecting groups; for example, ester such as acetyl and benzoyl, ether such as benzyl, and silyl ether such as tert-butyldimethylsilyl and tert-butyldiphenylsilyl.

### Reaction

A reaction in the process of the present invention takes the reaction scheme of the Mitsunobu reaction. The Mitsunobu reaction is a reaction named after its discoverer. Specifically, the reaction of optically-active secondary alcohol with, for example, diethyl azodicarboxylate (DEAD) and triphenylphosphine (PhP₃), and subsequently with benzoic acid produces a corresponding benzoyloxy derivative with inversion of configuration. Subsequent alkali hydrolysis allows for the conversion of the benzoyloxy derivative to corresponding alcohol. The Mitsunobu reaction proceeds with inversion of configuration, under a mild condition, and as such, is used for synthesizing many natural compounds. The use of alkyl halide instead of carboxylic acid that serves as a nucleophilic reagent gives a halogen compound and, similarly, the use of thiol gives thioether, and the use of imide or azide gives an amine derivative.

Although the production process of the present invention takes the scheme of the Mitsunobu reaction with inversion of configuration of a raw material, modified versions thereof can also be used in the process of the present invention. There have been reports on a variety of modified versions for the purpose of solving a problem such as by-products that hinder the purification of products or limitations on the range of available acid components, and any modified version well known by those skilled in the art can be used.

Any of organic solvents for dissolving isovitexin and vitexin or their compounds protected by protecting groups that are used as raw materials can be employed as long as it allows for the dissolution of the raw material. Specifically, benzene, toluene, THF, and xylene are preferred.

A reaction in the production process of the present invention is carried out in the presence of a compound represented by the formula (2):

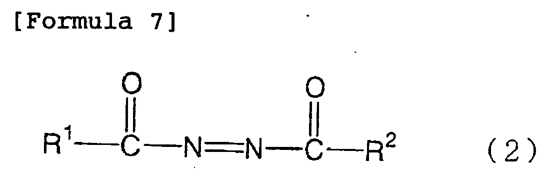

wherein R¹ and R² each are OR⁴ or N(R⁴)₂, and R⁴ is C₁ to C₄ alkyl or phenyl, and a compound represented by the formula (3):

[Formula 8]

(R³)₃P (3)

wherein R³ is C₁ to C₄ alkyl or phenyl. Specifically, compounds represented by the formula (2) include 1,1'-azobis(N,N'-dimethylformamide) and diethyl azodicarboxylate, and compounds represented by the formula (3) include tri-n-butylphosphine and triphenylphosphine. These compounds are commercially available.

The compound represented by the formula (2) and the compound represented by the formula (3) are added to a solution in which a raw material is dissolved in a solvent. It is preferred to add the compound represented by the formula (3) while cooling with ice.

Although the amounts of a raw material, and the compound represented by the formula (2) and the compound represented by the formula (3) to be added may be 1 or more equivalent(s) of the compound represented by the formula (2) and the compound represented by the formula (3) with respect to the raw material, it is preferred to add 2 or more equivalents of the compound represented by the formula (2) and the compound represented by the formula (3) with respect to the raw material.

A reaction temperature is preferably, but not particularly limited to, from room temperature to approximately 80°C. Although reaction time varies depending on a raw material used, the types and amounts of the compound represented by the general formula (2) and the compound represented by the formula (3), a solvent, and the like, it is preferably from 5 to 72 hours under a stirring condition.

According to the above-mentioned production process, the flavone C-glycoside derivative represented by the formula (1) or the salt thereof and the flavone C-glycoside derivative represented by the formula (4) or the salt thereof can be obtained.

It is noted that isovitexin and vitexin or their compounds protected by protecting groups that are unreacted raw materials can be collected and recycled for use in the subsequent cycles of the reaction.
Product
The production of the flavone C-glycoside derivative represented by the formula (1) or the salt thereof and the flavone C-glycoside derivative represented by the formula (4) or the salt thereof can be carried out by a separation/purification method well known by those skilled in the art, and be confirmed by, for example, structural analysis by means of spectrum analysis such as NMR or MS, and X-ray analysis.
Utility of product
The flavone C-glycoside derivative represented by the formula (1) or the salt thereof and the flavone C-glycoside derivative represented by the formula (4) or the salt thereof that are products according to the production processes of the present invention show an anti-allergic effect, and can therefore be employed for various uses such as medicines, foods, and cosmetics. In terms of medicines, the flavone C-glycoside derivative represented by the formula (1) or the salt thereof and/or the flavone C-glycoside derivative represented by the formula (4) or the salt thereof have an inhibitory effect on the formation of auricular edema induced by DNFB and an inhibitory effect on histamine release, and can therefore be used as anti-allergic drugs, especially for the purpose of treating atopic dermatitis, contact dermatitis, or pollinosis. In terms of foods, the flavone C-glycoside derivative represented by the formula (1) or the salt thereof and/or the flavone C-glycoside derivative represented by the formula (4) or the salt thereof can be blended as food additives into foods for specified health use (Tokutei Hokenyo Shokuhin), special nutritive foods, dietary supplements, health foods, or the like, for the purpose of alleviating or preventing symptoms of atopic dermatitis, contact dermatitis, or pollinosis. Foods to be added therewith can be various foods. In terms of Drinks the flavone C-glycoside derivative represented by the formula (1) or the salt thereof and/or the flavone C-glycoside derivative represented by the formula (4) or the salt thereof can be blended include drinks as foods for specified health use, special nutritive foods, or dietary supplements, or other nutritive drinks, health drinks, various health teas, or other teas. Other foods include confectionary, bread, noodles, processed soybean products, dairy products, processed egg products, fish cake, oils and fats, and seasonings. In terms of cosmetics, the compound or the salt thereof represented by the formula (1) and/or the compound or the salt thereof represented by the formula (4) can be added to skin-care products, foundations, makeup products, or the like, for the purpose of alleviating or preventing symptoms of atopic dermatitis, contact dermatitis, or pollinosis.

In terms of medicines, the compound or the salt thereof represented by the formula (1) and/or the compound or the salt thereof represented by the formula (4) can be administered orally either directly or after dilution with water or the like. Alternatively, the compound or the salt thereof represented by the formula (1) and/or the compound or the salt thereof represented by the formula (4) is formulated into a preparation with a pharmaceutical carrier known in the art. For example, the compound or the salt thereof represented by the formula (1) and/or the compound or the salt thereof represented by the formula (4) is processed into an oral liquid preparation such as a syrup, an extract, a powder, or the like, and blended with a pharmaceutically acceptable carrier. The resulting mixture can be administered as an oral solid preparation such as a tablet, a capsule, a granule, or a powder. The pharmaceutically acceptable carrier used is any of a variety of organic or inorganic carrier substances commonly used as raw materials for preparations, and is blended as, for example, an excipient, a lubricant, a binder, or a disintegrator in a solid preparation, or a solvent, an excipient, a suspension, or a binder in a liquid preparation. Preparation additives such as antiseptics, antioxidants, tinctions, and edulcorants can also be used if necessary.

Although the dosage form depends on the types and severity of diseases, examples thereof can include those suitable for oral, parenteral, or intranasal administration such as tablets or sugar-coated tablets, sublingual tablets, gelatin capsules, troches, suppositories, creams, ointments, or dermatologic gels. The effective dosage varies depending on the age and body weight of a patient, the type and severity of a disease, and an administration route. The dosage unit generally ranges from 0.0001 to 100 mg/kg per treatment with 1 to 3 dose(s) given for 24 hours.

In terms of foods, the compound or the salt thereof represented by the formula (1) and/or the compound or the salt thereof represented by the formula (4) can be administered in an original form or in the form of foods and drinks after being processed into an extract, a powder, or the like. Alternatively, the compound or the salt thereof represented by the formula (1) and/or the compound or the salt thereof represented by the formula (4) can be blended with a raw material for foods and drinks and an acceptable carrier to manufacture foods and drinks, which are generally used, and be provided as drinks, confectionary, bread, noodles, processed soybean products, dairy products, processed egg products, fish cake, oils and fats, seasoning, or the like.

Although the present invention will be described in more detail by Experiments and Examples, the scope of the present invention is not intended to be limited to such

### examples.

### EXAMPLES

Although the present invention will be described in more detail by Examples, the scope of the present invention is not intended to be limited to such examples.

### Example 1

To THF, 432 mg of isovitexin, 344 mg of 1,1'-azobis(N,N'-dimethylformamide), and 406 mg of tri-n-butylphosphine were added while cooling with ice and reacted with stirring at 70°C for 24 hours.

The reaction product was dissolved in methanol and separated/purified by gel filtration, followed by structural analysis. Table 1 shows NMR data. The product was identified as a compound represented by the formula (1) that was a compound in which OH at position 7 of isovitexin and OH at position 2 of the sugar chain were inverted, followed by intramolecular water-eliminating condensation. The yield was 50%.

**[Table 1]**

| | Example 1 (ppm) | Example 2 (ppm) | | Example 1 (ppm) | Example 2 (ppm) |
|---|---|---|---|---|---|
| C2 | 168.1 | 166.8 | | | |
| C3 | 102.9 | 104.3 | H3 | 6.58 | 6.66 |
| C4 | 184.8 | 184.4 | | | |
| C4a | 107.1 | 106.7 | | | |
| C5 | 166 | 166.2 | | | |
| C6 | 112.5 | 96.3 | | | |
| C7 | 168.4 | 168.9 | H6 | | 6.39 |
| C8 | 91.9 | 108.5 | H8 | 6.64 | |
| C8a | 161.2 | 155.1 | | | |
| C1' | 123 | 122.6 | | | |
| C2' C6' | 130.2 | 130.2 | H2' H6' | 7.81 | 7.9 |
| C3' C5' | 119.3 | 118 | H3' H5' | 6.8 | 6.93 |
| C4' | 165 | 164.6 | | | |
| C1" | 74.6 | 75.1 | H1" | 5.21 | 5.47 |
| C2" | 87 | 89.1 | H2" | 4.63 | 4.78 |
| C3" | 73.8 | 73.8 | H3" | 3.98 | 4.05 |
| C4" | 69.8 | 69.8 | H4" | 3.58 | 3.62 |
| C5" | 80.6 | 80.5 | H5" | 3.33 | 3.47 |
| C6" | 63.5 | 63.4 | H6"a | 3.62 | 3.66 |
| | | | H6"b | 3.86 | 3.87 |

### Example 2

To THF, 432 mg of vitexin, 344 mg of 1,1'-azobis(N,N'-dimethylformamide), and 406 mg of tri-n-butylphosphine were added while cooling with ice and reacted with stirring at 70°C for 24 hours.

The reaction product was dissolved in methanol and separated/purified by gel filtration, followed by structural analysis. Table 1 shows NMR data. The product was identified as a compound represented by the formula (4) that was a compound in which OH at position 7 of vitexin and OH at position 2 of the sugar chain were inverted, followed by intramolecular water-eliminating condensation. The yield was 50%.

### Example 3

To benzene, 432 mg of isovitexin, 344 mg of 1,1'-azobis(N,N'-dimethylformamide), and 406 mg of tri-n-butylphosphine were added while cooling with ice and reacted with stirring at 70°C for 24 hours.

The reaction product was dissolved in methanol and separated/purified by gel filtration, followed by structural analysis. Table 2 shows NMR data. The product was identified as a compound represented by the formula (1) that was a compound in which OH at position 7 of isovitexin and OH at position 2 of the sugar chain were inverted, followed by intramolecular water-eliminating condensation. The yield was 10%.

**[Table 2]**

| | Example 3 (ppm) | Example 4 (ppm) | | Example 3 (ppm) | Example 4 (ppm) |
|---|---|---|---|---|---|
| C2 | 168.1 | 166.8 | | | |
| C3 | 102.9 | 104.3 | H3 | 6.58 | 6.66 |
| C4 | 184.8 | 184.4 | | | |
| C4a | 107.1 | 106.7 | | | |
| C5 | 166 | 166.2 | | | |
| C6 | 112.5 | 96.3 | H6 | | 6.39 |
| C7 | 168.4 | 68.9 | | | |
| C8 | 91.9 | 108.5 | H8 | 6.64 | |
| C8a | 161.2 | 155.1 | | | |
| C1' | 123 | 122.6 | | | |
| C2' C6' | 130.2 | 130.2 | H2' H6' | 7.81 | 7.9 |
| C3' C5' | 119.3 | 118 | H3' H5' | 6.8 | 6.93 |
| C4' | 165 | 164.6 | | | |
| C1" | 74.6 | 75.1 | H1" | 5.21 | 5.47 |
| C2" | 87 | 89.1 | H2" | 4.63 | 4.78 |
| C3" | 73.8 | 73.8 | H3" | 3.98 | 4.05 |
| C4" | 69.8 | 69.8 | H4" | 3.58 | 3.62 |
| C5" | 80.6 | 80.5 | H5" | 3.33 | 3.47 |
| C6" | 63.5 | 63.4 | H6"a | 3.62 | 3.66 |
| | | | H6"b | 3.86 | 3.87 |

### Example 4

To benzene, 432 mg of vitexin, 344 mg of 1,1'-azobis(N,N'-dimethylformamide), and 406 mg of tri-n-butylphosphine were added while cooling with ice and reacted with stirring at 70°C for 24 hours.

The reaction product was dissolved in methanol and separated/purified by gel filtration, followed by structural analysis. Table 2 shows NMR data. The product was identified as a compound represented by the formula (4) that was a compound in which OH at position 7 of vitexin and OH at position 2 of the sugar chain were inverted, followed by intramolecular water-eliminating condensation. The yield was 10%.

### INDUSTRIAL APPLICABILITY

A flavone C-glycoside derivative represented by the formula (1) or a salt thereof and a flavone C-glycoside derivative represented by the formula (4) or a salt thereof that are substances having an anti-allergic effect can efficiently be obtained. Particularly, processes of the present invention are characterized in that the flavone C-glycoside derivative represented by the formula (1) or the salt thereof and the flavone C-glycoside derivative represented by the formula (4) or the salt thereof can efficiently be obtained by convenient processes with easily-available raw materials.

## Claims

1. A process of producing a flavone C-glycoside derivative represented by the formula (1): 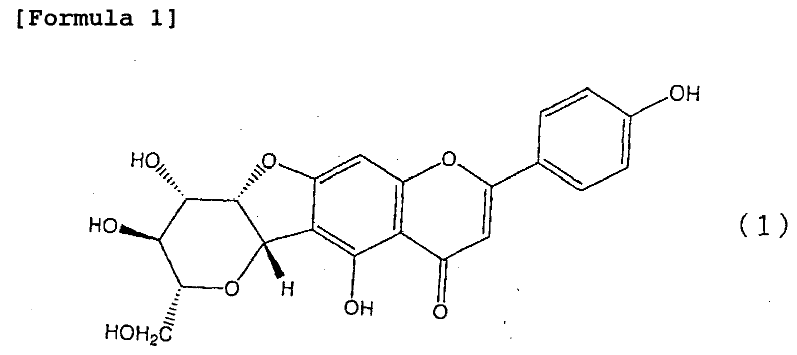 or a salt thereof comprising the step of reacting isovitexin used as a raw material in an organic solvent in the presence of a compound represented by the formula (2): 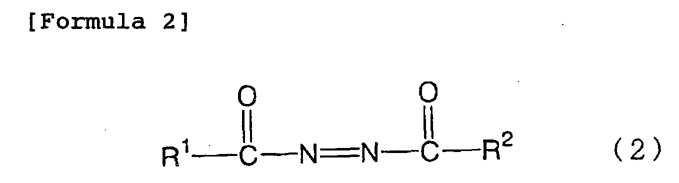 wherein R¹ and R² each are OR⁴ or N(R⁴)₂, and R⁴ is C₁ to C₄ alkyl or phenyl, and a compound represented by the formula (3):
[Formula 3] (R³)₃P (3)
wherein R³ is C₁ to C₄ alkyl or phenyl.

2. A process of producing a flavone C-glycoside derivative represented by the formula (4): 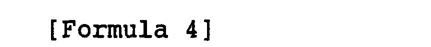 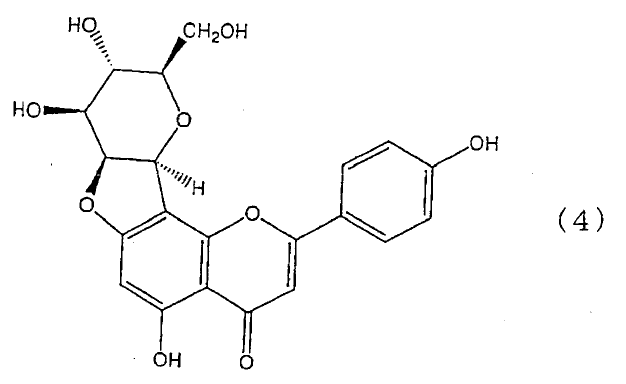 or a salt thereof comprising the step of reacting vitexin used as a raw material in an organic solvent in the presence of a compound represented by the formula (2): 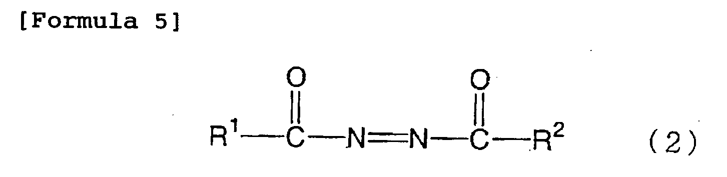 wherein R¹ and R² each are OR⁴ or N(R⁴)₂, and R⁴ is C₁ to C₄ alkyl or phenyl, and a compound represented by the formula (3):
[Formula 6] (R³)₃P (3)
wherein R³ is C₁ to C₄ alkyl or phenyl.

3. The production process according to claim 1 or claim 2, wherein the organic solvent is selected from the group consisting of benzene, toluene, THF, and xylene.

4. The production process according to claim 3, wherein the organic solvent is THF.

5. The production process according to claim 1 or claim 2, wherein the compound represented by the formula (2) is 1,1'-azobis(N,N'-dimethylformamide).

6. The production process according to claim 1 or claim 2, wherein the compound represented by the formula (3) is tri-n-butylphosphine.

7. The production process according to claim 1 or claim 2, wherein the compound represented by the formula (2) is 1,1'-azobis(N,N'-dimethylformamide) and the compound represented by the formula (3) is tri-n-butylphosphine.

8. The production process according to claim 1, wherein the isovitexin is protected by a protecting group.

9. The production process according to claim 2, wherein the vitexin is protected by a protecting group.

10. The production process according to claim 1, wherein the yield of the flavone C-glycoside derivative represented by the formula (1) or the salt thereof is 40% or more.

11. The production process according to claim 2, wherein the yield of the flavone C-glycoside derivative represented by the formula (4) or the salt thereof is 40% or more.

12. The production process according to claim 5, wherein the compound represented by the formula (3) is supported on a styrene resin.

13. The production process according to claim 1, wherein unreacted isovitexin is recycled.

14. The production process according to claim 2, wherein unreacted vitexin is recycled.
